# EUROPEAN PATENT APPLICATION

(11) **EP 4 159 719 A1**
(43) Date of publication of application: **05.04.2023**
(21) Application number: 21812542.5
(22) Date of filing: 26.05.2021
(51) Int. Cl.: C07D 213/127

(54) **PREPARATION METHOD FOR (S)-2-AMINO-3-(4-(2,3-DIMETHYLPYRIDIN-4-YL)PHENYL)METHYL PROPIONATE DIACID SALT**

(30) Priority: 28.05.2020 CN 202010481657
(71) Applicant: Hangzhou Zhongmeihuadong Pharmaceutical Co., Ltd., GongShu District HangZhou, Zhejiang 310011 (CN)
(72) Inventor: HU, Haiwen, HangZhou, Zhejiang 310011 (CN); HU, Fan, HangZhou, Zhejiang 310011 (CN); ZHOU, Yubao, HangZhou, Zhejiang 310011 (CN)
(74) Representative: Mewburn Ellis LLP
(86) International application number: PCT/CN2021/095965
(87) International publication number: WO 2021/238963

(57) **Abstract**

The invention provides a method for preparing a key intermediate for (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid dihydrochloride, Methyl (S)-2-amino-3-[4-(2,3-dimethylpyridin-4-yl)-phenyl]-propionate diacid salt. Compared with the prior art, the method does not require column chromatographic separation and purification, has the advantages of low cost and high yield, and is suitable for industrial production.

## Description

### Technical Field

The invention belongs to the technical field of drug synthesis, and particularly relates to a method for preparing a key intermediate for (S)-2-(3S,8S)-3-(4-(3,4-dichlorobenzyloxy)phenyl-7-((S)-1-phenylpropyl)-2,3,6,7,8,9-hexahydro-[1,4] -dioxino[2,3-g]isoquinolin-8-ylformylamino)-3-(4-(2,3-dimethylpyridin-4-yl)phenyl)propionic acid dihydrochloride, Methyl (S)-2-amino-3-(4-(2,3-dimethylpyridin-4-yl)phenyl)propionate diacid salt.

### Background of the Invention

(S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid dihydrochloride (compound A) is a small molecule, non-peptide glucagon-like peptide 1 (GLP-1) receptor agonist. It has a chemical name of (S)-2-(3S,8S)-3-(4-(3,4-dichlorobenzyloxy)phenyl-7-((S)-1-phenylpropyl)-2,3,6,7,8,9-hexahydro-[1,4] -dioxino[2,3-g]isoquinolin-8-ylformylamino)-3-(4-(2,3-dimethylpyridin-4-yl)phenyl)propionic acid dihydrochloride, molecular formula of C₅₀H₄₉Cl₄N₃O₆, molecular weight of 929.76, and the following chemical structure: 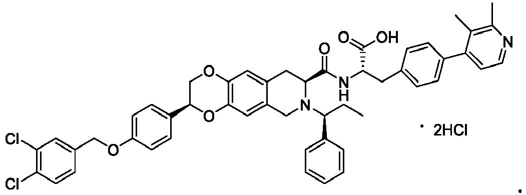 compound A has four chiral centers, among which, methyl (S)-2-amino-3-[4-(2,3-dimethylpyridin-4-yl)-phenyl]-propionate diacid salt introduces the last chiral center for the synthesis of TTP273 and thus is a key intermediate for the synthesis of compound A.

CN102378574B discloses a method for the synthesis of free base compound A, and particularly discloses a 3-step reaction route for preparing methyl (S)-2-amino-3-[4-(2,3-dimethylpyridin-4-yl)-phenyl]-propionate dihydrochloride (IV), which utilizes methyl (S)-3-(4-bromo-phenyl)-2-tert-butoxycarbonylamino-propionate VI as the starting material and comprises 3 steps including Suzuki coupling and deprotection:

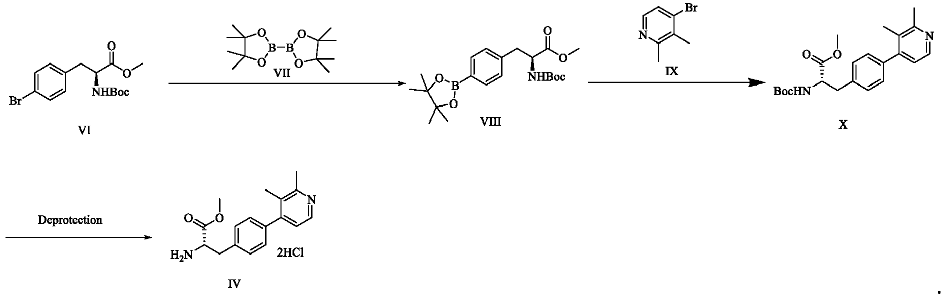

More particularly, the prior art discloses the following reaction steps: adding bis(pinacolato)diboron (VII) to compound VI, stirring under the protection of nitrogen at 75°C for 3 hours, and purifying by using chromatographic column to give methyl (S)-2-tert-butoxycarbonylamino-3-[4-(4,4,5,5-tetramethyl-[1,3,2]dioxacyclopentaboran -2-yl)-phenyl]-propionate (VIII); adding 4-bromo-2,3-dimethylpyridine (IX), stirring under the protection of nitrogen at 80°C for 18 hours, purifying by using chromatographic column to give methyl (S)-2-tert-butoxycarbonylamino-3-[4-(2,3-dimethylpyridin-4-yl)-phenyl]-propionate (X), and at last removing the protective group to give methyl (S)-2-amino-3-[4-(2,3-dimethylpyridin-4-yl)-phenyl]-propionate dihydrochloride (IV).

The above reaction route requires multiple column chromatographic separations and purifications, and has a total yield of only 49%; moreover, the starting materials, compound VI and compound IX are expensive, the researchers have found that compound IV is highly hygroscopic and easily to be deteriorated, it is difficult to obtain solid compound IV through conventional solvent crystallization, hence it is difficult to store,transport and industrial produce compound IV.

Therefore, it is necessary to optimize the preparation method of the key intermediate for compound A, methyl (S)-2-amino-3-[4-(2,3-dimethylpyridin-4-yl)-phenyl]-propionate diacid salt, looking for replaceable salt forms, increase the yield, and lower the cost, so as to apply to industrial production.

### Summary of the Invention

The invention provides a method for preparing a key intermediate of compound A, methyl (S)-2-amino-3-[4-(2,3-dimethylpyridin-4-yl)-phenyl]-propionate diacid salt. Compared with the prior art, the method does not require column chromatographic separation and purification, has the advantages of low cost and high yield, and is suitable for industrial production.

The preparation method of the invention is as follows: 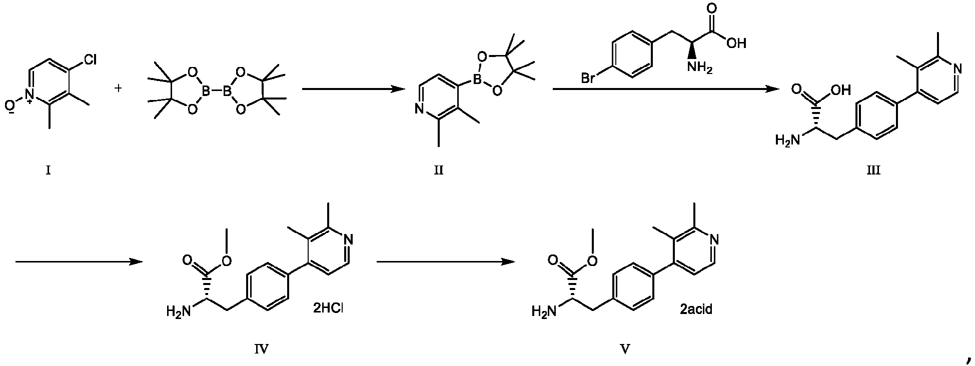
step (a): reacting compound I with bis(pinacolato)diboron to give compound II;
step (b): reacting compound II with 4-bromo-L-phenylalanine to give compound III;
step (c): subjecting compound III to esterification to give compound IV.

Alternatively, the method further comprises step (d): after salt dissociation of compound IV, mixing it in free form with organic acid or inorganic acid to form salt and then give compound V after purification.

As a specific embodiment, step (a) is carried out under the protection of nitrogen.

As a specific embodiment, in step (a), compound I and organic acid salt are first added into the solvent, and the temperature of the mixture is maintained in the range of -10^{∼}10°C; wherein the organic acid salt is one, two or more selected from the group consisting of potassium acetate, sodium acetate, potassium oxalate, sodium oxalate, sodium citrate, potassium citrate, L-potassium tartrate, L-sodium tartrate, potassium malate, sodium malate, potassium succinate, sodium succinate, potassium maleate, and sodium maleate, and preferably potassium acetate; and the solvent is one, two or more selected from the group consisting of xylene, toluene or chlorobenzene.

As a specific embodiment, in step (a), bis(pinacolato)diboron is added in two portions; the first portion is added at a temperature of -10^{~}10°C, and the second portion is added at a temperature of 20^{~}30°C.

As a specific embodiment, in step (a), after the completion of the second reaction of bis(pinacolato)diboron, catalyst A is added; and the catalyst A is palladium catalyst alone or a mixed system of palladium catalyst and organophosphorus ligand, wherein the palladium catalyst is selected from the group consisting of Pd₂(dba)₃, PdCl₂(PPh₃)₂, and Pd(OAc)₂, and the organophosphorus ligand is one, two or more selected from the group consisting of PCy₃, PPh₃, n-Bu₃P, and P(OMe)₃, and preferably a mixed system of Pd₂(dba)₃ and PCy₃.

As a specific embodiment, in step (a), the reaction temperature after the addition of the catalyst is in the range of 100^{~}135°C, and preferably in the range of 110^{~}120°C.

As a specific embodiment, in step (a), compound I, organic acid salt and bis(pinacolato)diboron are fed in a molar ratio in the range of 1:2:2^{~}1:4:3, and the molar amount of the catalyst is 0.1^{~}1% of that of compound I.

As a specific embodiment, the workup mode of step (a) is as follows: adding heptane to dilute, stirring the mixture, filtering out the insoluble substance, extracting the filtrate with diluted hydrochloric acid, washing the obtained aqueous layer with dichloromethane or ethyl acetate, removing out the organic layer, concentrating to a volume 2^{~}5 times to the volume of compound I, and giving compound II.

As a specific embodiment, in step (a), the concentration of the diluted hydrochloric acid used in the workup is 1^{~}2mol/L.

As a specific embodiment, step (b) is carried out under the protection of nitrogen.

As a specific embodiment, in step (b), water and organic solvent are added to the concentrated solution of compound II, and alkaline reagent A is added to adjust pH to approximate 7; wherein the organic solvent is one, two or more selected from the group consisting of ethanol, n-propanol, n-butanol, tetrahydrofuran, 1,4-dioxane, toluene, and xylene, and preferably ethanol; the alkaline reagent A is one, two or more selected from the group consisting of sodium carbonate, potassium carbonate, cesium carbonate, and lithium carbonate, and preferably sodium carbonate; and 4-bromo-L-phenylalanine and compound II are fed at a ratio in the range of 1:1.5^{~}1:2.5, and preferably 1:2.

As a specific embodiment, in step (b), after the adjustment of pH, alkaline reagent B and 4-bromo-L-phenylalanine are successively added, and the reaction temperature is adjusted to 30^{~}40°C; wherein the alkaline reagent is one, two or more selected from the group consisting of sodium carbonate, potassium carbonate, cesium carbonate, lithium carbonate, barium hydroxide, and potassium phosphate, and preferably sodium carbonate.

As a specific embodiment, in step (b), catalyst B is added, and the catalyst B is a palladium catalyst alone or a mixed system of palladium catalyst and organophosphorus ligand, wherein the palladium catalyst is selected from the group consisting of Pd(OAc)₂, Pd₂(dba)₃, PdCl₂(PPh₃)₂, PdCl₂dppf, and Pd(PPh₃)₄, and the organophosphorus ligand is one, two or more selected from the group consisting of Ph₂P(CH₂)₂PPh₂(dppe), Ph₂P(CH₂)₃PPh₂(dppp), PCy₃, n-Bu₃P, P(OMe)₃, and PPh₃, and preferably a mixed system of Pd₂(dba)₃ and PCy₃; wherein the molar amount of the catalyst is 1^{~}5% of that of 4-bromo-L-phenylalanine; and the reaction temperature is in the range of 60^{~}90°C, and preferably in the range of 75^{~}85°C.

As a specific embodiment, the workup mode of step (b) is as follows: concentrating under reduced pressure, evaporating off a certain part of solvent that is 4 times in volume to the volume of 4-bromo-L-phenylalanine, further adding purified water to make up to the original volume, extracting with ethyl acetate or dichloromethane, and separating and removing out the organic phase; dropwise adding acid to the aqueous phase to adjust pH to 1^{~}2, and filtering; extracting the filtrate with ethyl acetate or dichloromethane, separating and removing out the organic phase, adding sodium hydroxide aqueous solution to the aqueous phase to adjust pH to 5^{~}8, stirring to crystalize, and giving compound III; the acid used to adjust pH is one of hydrochloric acid, sulfuric acid, and phosphoric acid, and preferably hydrochloric acid.

As a specific embodiment, in step (c), methanol serves as both solvent and reaction reagent, and optionally, oxalyl chloride or thionyl chloride is added, and the reaction temperature is controlled in the range of 40^{~}70°C, and preferably 55^{~}65°C.

As a specific embodiment, in step (c), after the completion of the reaction, the reaction mixture is concentrated to remove the solvent and give compound IV.

As a specific embodiment, in step (d), an alkaline aqueous solution is added to the product of step (c) to adjust pH, then the mixture is extracted with solvent 1 to 3 times, aqueous phase is separated and removed out, and organic phase is concentrated to give free base of compound IV; wherein the extraction solvent is one selected from the group consisting of ethyl acetate, dichloromethane, isopropyl acetate, butyl acetate or 2-methyltetrahydrofuran.

As a specific embodiment, in step (d), the solution of the free base of compound IV is mixed homogeneously with solvent, and then a solution of organic acid or inorganic acid is added; the mixture is stirred to form salt and crystalize, and then compound V is obtained.

As a specific embodiment, in step (d), the solvent is one, two or more selected from the group consisting of dichloromethane, acetone, isopropanol, acetonitrile, and tetrahydrofuran, the organic acid or inorganic acid is one selected from the group consisting of oxalic acid, L-tartaric acid, malic acid, succinic acid, maleic acid, citric acid, phosphoric acid, sulfuric acid, and hydrobromic acid, and preferably oxalic acid or phosphoric acid; and the organic acid or inorganic acid is dissolved in acetone, methanol or ethanol.

As a specific embodiment, the methyl (S)-2-amino-3-[4-(2,3-dimethylpyridin-4-yl)-phenyl]-propionate diacid salt obtained through steps (a) to (c) or through steps (a) to (d) is a key intermediate for the preparation of compound A.

The term "diacid salt" refers to a 1:2 molar ratio of free base to acid, the acids including organic and inorganic acids.

Compared with the prior art, the preparation method of the invention for preparing methyl (S)-2-amino-3-[4-(2,3-dimethylpyridin-4-yl)-phenyl]-propionate diacid salt has the advantages of low cost of raw materials and high yield of the product, does not require column chromatographic purification, and is more suitable for industrial production. Moreover, the methyl (S)-2-amino-3-[4-(2,3-dimethylpyridin-4-yl)-phenyl]-propionate diacid salt that is further prepared from methyl (S)-2-amino-3-[4-(2,3-dimethylpyridin-4-yl)-phenyl]-propionate dihydrochloride has better storage stability.

### Detailed Description of the Invention

The invention will be further illustrated by combining the following specific examples. The following examples are used to explain the method of the invention and the core concept thereof, and for those skilled in the art, any possible change or substitution without departing from the inventive concept will fall within the protection scope of the invention. In the following examples, where the specific conditions of the experimental methods are not indicated, they are typically the conventional conditions, or are those recommended by the raw material or commodity manufactures; and the solvents without indicating the source are typically conventional solvents that are commercially available.

The detection instrument used in the present invention is:
1. NMR
   Instrument model: Bruker DMX-500
2. Mass Spectrometer
   Instrument model: Agilent 6460, test conditions: ESI source

The amount of materials fed in Examples 1-4 is shown in Table 1.

**Table 1 Amount of materials fed in Examples 1-4**

| step | Name of the materials | Example 1 | Example 2 | Example 3 | Example 4 |
|---|---|---|---|---|---|
| | | unit: g | unit: g | unit: g | unit: kg |
| a | compound I | 15.76 | 34.36 | 26.00 | 22.46 |
| | potassium acetate | 19.63 | 64.18 | 64.77 | 42.00 |
| | bis(pinacolato)diboron | 50.70 | 138.15 | 125.48 | 90.31 |
| | xylene or toluene or chlorobenzene | 106.12 | 215.97 | 170.21 | 148.90 |
| | Pd₂(dba)₃ | 0.1 | 1.00 | 1.2 | 0.65 |
| | PCy₃ | 0.25 | 0.31 | 0.1 | 0.20 |
| | n-heptane | 260.30 | 582.56 | 435.98 | 372.35 |
| | concentrated hydrochloric acid | 12.51 | 22.15 | 18.70 | 16.84 |
| | dichloromethane or ethyl acetate | 14.56 | 35.05 | 26.04 | 22.46 |
| b | compound II (pH=2) | 23.17 | 50.50 | 36.54 | 33.01 |
| | sodium carbonate | 21.87 | 49.00 | 38.15 | 32.68 |
| | ethanol or n-propanol or tetrahydrofuran | 60.21 | 130.84 | 95.17 | 83.85 |
| | 4-bromo-L-phenylalanine | 16.17 | 26.07 | 15.03 | 17.28 |
| | Pd₂(dba)₃/PCy₃ or PdCl₂(PPh₃)₂ | 0.95 | 3 | 1.7 | 1.35 |
| | ethyl acetate or dichloromethane | 194.23 | 432.50 | 304.87 | 279.62 |
| | concentrated hydrochloric acid | 40.15 | 79.25 | 63.40 | 55.79 |
| | sodium hydroxide | 8.98 | 20.63 | 16.01 | 13.87 |
| | acetonitrile | 67.01 | 145.98 | 108.70 | 93.10 |
| c and d | compound III | 15.06 | 24.54 | 14.32 | 16.84 |
| | methanol | 162.75 | 288.17 | 158.15 | 186.38 |
| | oxalyl chloride or thionyl chloride | 13.21 | 26.12 | 14.35 | 16.84 |
| | sodium carbonate | 17.17 | 27.41 | 16.48 | 18.69 |
| | ethyl acetate or dichloromethane or 2-methyltetrahydrofuran | 194.46 | 345.51 | 194.81 | 223.59 |
| | oxalic acid or phosphoric acid | 16.82 | 25.50 | 15.5 | 16.84 |
| | acetone or methanol or ethanol | 137.57 | 230.61 | 126.94 | 149.01 |

### Example 1

(a) under the protection of inert gas, xylene and potassium acetate were successively added to compound I. The above mixture was cooled down to approximate 0°C, and then bis(pinacolato)diboron was added. The mixture was stirred at approximate 0°C for 1 hour and then maintained at approximate 0°C, and bis(pinacolato)diboron was added. The above mixture was stirred at approximate 15°C for 1 hour. The resultant reaction mixture was further stirred at approximate 20°C until the reaction was complete. Pd₂(dba)₃ and PCy₃ were added, and the mixture was heated to a temperature of in the range of 110^{~}120°C and reacted for 8 hours. After the completion of the reaction, the mixture was cooled down, and heptane was added to dilute the mixture and then stirred for 0.5 hour. The unsoluble substance of the reaction mixture was filtered out, and the filtrate was extracted with diluted HCl. The aqueous layer was washed with dichloromethane, and the organic layer was removed out. The solution was concentrated to a volume 2.5 times to the volume of compound I, to give compound II (99% yield).
(b) under the protection of inert gas, water and ethanol were added to the solution of compound II, and then sodium carbonate was added to the reaction mixture to adjust pH to approximate 7. Subsequently, sodium carbonate and 4-bromo-L-phenylalanine were successively added to the above reaction mixture. The reaction mixture is heated to a temperature in the range of 30^{~}40°C, Pd₂(dba)₃ and PCy₃ were added in a nitrogen atmosphere, and the mixture was heated to approximate 80°C to react for 12 hours. After the completion of the reaction, the reaction mixture was concentrated under reduced pressure to evaporate off a certain part of solvent that is 4 times in volume to the volume of 4-bromo-L-phenylalanine, and then water was added to make up to the original volume. Dichloromethane was added to extract three times, the organic phase was separated and removed out, and concentrated hydrochloric acid was dropwise added to the aqueous phase to adjust pH to 1^{~}2. After filtration, the filtrate was further extracted with dichloromethane once, the organic phase was separated and removed out, and sodium hydroxide aqueous solution was added to the aqueous phase to adjust pH to approximate 7. The mixture was stirred to crystalize, to give compound III (84% yield).
(c) Compound III was added into methanol, and the mixture was cooled down to 0°C. Oxalyl chloride was dropwise added to the above cooled mixture, and then the reaction mixture was heated to approximate 60°C to react for 1.5 hours. After the completion of the reaction, the reaction mixture was concentrated to remove off solvent, to give compound IV.
(d) Compound IV was added into purified water and dissolved completely. At a temperature controlled below 20°C, sodium carbonate aqueous solution was dropwise added to adjust pH to approximate 8. Then, dichloromethane was added to extract three times, the aqueous phase is separated and removed out. The organic phase was cooled down to below 5°C, and a solution of oxalic acid in acetone was dropwise added. The mixture was stirred to crystalize, to give compound V (80% yield). 1H NMR (500 MHz, d6-DMSO) δ 8.37 (d, J=5.0Hz, 1H), 7.38-7.16 (m, 4H), 7.15 (s, 1H), 4.35-4.32 (m, 1H), 3.26-3.15 (m, 2H), 3.70 (s, 3H), 2.56 (s, 3H), 2.53 (s, 3H); ¹³C NMR (100 MHz, d6-DMSO) δ 169.5, 163.4(4C), 158.5, 157.8, 148.9, 145.9, 142.0, 141.8, 140.9, 138.7, 156.6, 149.7, 144.1, 137.6, 134.8, 129.5(2C), 129.4, 128.9(2C), 122.6, 53.2, 52.6, 35.7, 22.2, 15.8; It is confirmed by carbon spectrum that the structure contains 2 molecules of oxalic acid, and the molecular formula is C₁₇H₂₀N₂O₂ ·2C₂H₂O₄. HRMS (ESI) Calcd. For C₁₇H₂₀N₂O₂: 285.1598 [M+H], found: 285.1604.

### Example 2

(a) under the protection of inert gas, toluene and potassium acetate were successively added to compound I. The above mixture was cooled down to approximate -5°C, and then bis(pinacolato)diboron was added. The mixture was stirred at approximate 0°C for 0.5 hour and then maintained at approximate 0°C, and then bis(pinacolato)diboron was added. The above mixture was stirred at approximate 10°C for 1.5 hour. The resultant reaction mixture was further stirred at 25°C for 10 hours until the reaction was complete. Then, Pd₂(dba)₃ and PCy₃ were added, and the reaction mixture was heated to a temperature in the range of 110^{~}120°C and reacted for 8 hours. After the completion of reaction, the reaction mixture was cooled down, and n-heptane was added to dilute the mixture and then stirred for 1 hour. The unsoluble substance in the reaction mixture was filtered off, and the filtrate was extracted with diluted HCl. The aqueous layer was washed with ethyl acetate, and the organic layer was removed out. The solution was concentrated to a volume three times to the volume of compound I to give compound II (98% yield).
(b) under the protection of inert gas, water and n-propanol were added to the solution of compound II, and then sodium carbonate was added to the reaction mixture to adjust pH to approximate 7. Subsequently, sodium carbonate and 4-bromo-L-phenylalanine were successively added to the above reaction mixture. The reaction mixture was heated to a temperature in the range of 30^{~}40°C, PdCl₂(PPh₃)₂ and PCy₃ were added in a nitrogen atmosphere, and then the mixture was heated to approximate 80°C and reacted for 14 hours. After the completion of reaction, the reaction mixture was concentrated under reduced pressure to evaporate off a certain part of solvent that is 4 times in volume to the volume of 4-bromo-L-phenylalanine, and then purified water was further added to make up to the original volume. Ethyl acetate was added to extract three times, the organic phase was separated and removed out, and concentrated hydrochloric acid was dropwise added to the aqueous phase to adjust pH to approximate 1.5. After filtration, the filtrate was further extracted with ethyl acetate once, the organic phase was separated and removed out, and 25% sodium hydroxide aqueous solution was added to the aqueous phase to adjust pH to 8. The mixture was stirred to crystalize, to give compound III (85% yield).
(c) Compound III was added into methanol and cooled down to 0°C. Thionyl chloride was dropwise added to the above cooled mixture, and then the reaction mixture was heated to approximate 60°C and reacted for 3 hours. After the completion of reaction, the reaction mixture was concentrated to remove off solvent, to give compound IV.
(d) Compound IV was added into purified water and stirred until complete dissolution. At a temperature controlled below 20°C, sodium carbonate aqueous solution was dropwise added to adjust pH to approximate 9. Then, ethyl acetate was added to extract three times, the aqueous phase is separated and removed out. The organic phase was cooled down to a temperature below 5°C and a solution of oxalic acid in methanol was dropwise added. The mixture was stirred to crystalize, to give compound V (81% yield). The characterization data of the obtained compound are the same as those in Example 1.

### Example 3

(a) under the protection of inert gas, chlorobenzene and potassium acetate were successively added to compound I. The above mixture was cooled down to approximate 0°C, and then bis(pinacolato)diboron was added. The mixture was stirred at approximate 0°C for 1 hour and maintained at approximate 0°C, and then bis(pinacolato)diboron was added. The above mixture was stirred at approximate 10°C for 1.5 hours. The resultant reaction mixture was further stirred at 30°C or 10 hours until the reaction was complete. Then, Pd₂(dba)₃ and PCy₃ were added, and the reaction mixture was heated to a temperature in the range of 110^{~}120°C and reacted for 8 hours. After the completion of reaction, the reaction mixture was cooled down to 70°C, and n-heptane was added to dilute the mixture and then stirred for 1 hour. The unsoluble substance in the reaction mixture was filtered off, and the filtrate was extracted with diluted HCl. The aqueous layer was washed with dichloromethane, and the organic layer was removed out. The solution was concentrated to a volume five times to the volume of compound I, to give compound II (95% yield).
(b) under the protection of inert gas, water and tetrahydrofuran were added to the solution of compound II, and then sodium carbonate was added to the reaction mixture to adjust pH to approximate 7. Subsequently, sodium carbonate and 4-bromo-L-phenylalanine were successively added to the above reaction mixture. The reaction mixture was heated to a temperature in the range of 30^{~}40°C, PdCl₂(PPh₃)₂ was added in a nitrogen atmosphere, and then the mixture was heated to approximate 80°C and reacted for 11 hours. After the completion of reaction, the mixture was concentrated under reduced pressure to evaporate off a certain part of solvent that is 4 times in volume to the volume of 4-bromo-L-phenylalanine, and then purified water was further added to make up to the original volume. Ethyl acetate was added to extract three times, the organic phase was separated and removed out, and concentrated hydrochloric acid was dropwise added to the aqueous phase to adjust pH to approximate 1.5. After filtration, the filtrate was further extracted with ethyl acetate once, the organic phase was separated and removed out, and sodium hydroxide aqueous solution was added to the aqueous phase to adjust pH to 7.5. The mixture was stirred to crystalize, to give compound III (86% yield).
(c) Compound III was added into methanol and cooled down to 0°C. Thionyl chloride was dropwise added to the above cooled mixture, and then the reaction mixture was heated to approximate 60°C and reacted for 1.5 hours. After the completion of reaction, the reaction mixture was concentrated to remove off solvent, to give compound IV.
(d) Compound IV was added into purified water and stirred until complete dissolution. At a temperature controlled below 20°C, 20% sodium carbonate aqueous solution was dropwise added to adjust pH to 8.5. Then, dichloromethane was added to extract three times, the aqueous phase is separated and removed out. The organic phase was concentrated to dryness, and acetone was added and stirred. The mixture was cooled down to a temperature below 5°C, and a solution of phosphoric acid in acetone was dropwise added. The mixture was stirred to crystalize, to give compound V (82% yield). ¹H NMR (500 MHz, d6-DMSO) δ 8.30 (d, J=5.0Hz, 1H), 7.36-7.30 (m, 4H), 7.04 (s, 1H), 4.25-4.23 (m, 1H), 3.24-3.11 (m, 2H), 3.68 (s, 3H), 2.51 (s, 3H), 2.16 (s, 3H); ¹³C NMR (100 MHz, d6-DMSO) δ 170.0, 157.3, 148.3, 145.4, 138.0, 134.7, 129.5(2C), 128.9(2C), 128.4, 122.1, 53.4, 52.5, 36.1, 23.0, 15.8; HRMS (ESI) Calcd. For C₁₇H₂₀N₂O₂:285.1598 [M+H], found: 285.1604_{∘}

With reference to the four general rules 3103 of the 2015 edition of the Chinese Pharmacopoeia, the phosphorus content was measured to be 12.3%, proves that it contains two molecules of phosphoric acid, that is, the molecular formula is C₁₇H₂₀N₂O₂ ·2H₃PO₄.

### Example 4

(a) under the protection of inert gas, xylene and potassium acetate were successively added to compound I. The above mixture was cooled down to approximate 0°C, and then bis(pinacolato)diboron was added. The mixture was stirred at approximate 0°C for 1 hour and maintained at approximate 0°C, and then bis(pinacolato)diboron was added. The above mixture was stirred at approximate 15°C for 1 hour. The resultant reaction mixture was further stirred at 25°C until the reaction was complete. Then, Pd₂(dba)₃ and PCy₃ were added, and the reaction mixture was heated to a temperature in the range of 110^{~}120°C and reacted. After the completion of reaction, the reaction mixture was cooled down to 70°C, and n-heptane was added to dilute the mixture and then stirred for 1 hour. The unsoluble substance in the reaction mixture was filtered off and washed with n-heptane, and the filtrate was extracted with diluted HCl. The aqueous layer was washed with dichloromethane, and the organic layer was removed out. The solution was concentrated to a volume three times to the volume of compound I, to give compound II (100% yield).
(b) under the protection of inert gas, water and n-propanol were added to the solution of compound II, and then sodium carbonate was added to the reaction mixture to adjust pH to approximate 7. Subsequently, sodium carbonate and 4-bromo-L-phenylalanine were successively added to the above reaction mixture. The reaction mixture was heated to a temperature in the range of 30^{~}40°C, PdCl₂(PPh₃)₂ was added in a nitrogen atmosphere, and the mixture was heated to approximate 80°C and reacted. After the completion of reaction, the mixture was concentrated under reduced pressure to evaporate off a certain part of solvent that is 4 times in volume to the volume of 4-bromo-L-phenylalanine, and then purified water was further added to make up to the original volume. Dichloromethane was added to extract three times, the organic phase was separated and removed out, and concentrated hydrochloric acid was dropwise added to the aqueous phase to adjust pH to 1-2. After filtration, the filtrate was further extracted with dichloromethane once, the organic phase was separated and removed out, and sodium hydroxide aqueous solution was added to the aqueous phase to adjust pH to approximate 6. The mixture was stirred to crystalize, to give compound III (85% yield).
(c) Compound III was added into methanol and cooled down to 0°C. Thionyl chloride was dropwise added to the above cooled mixture, and then the reaction mixture was heated to approximate 60°C and reacted for 3 hours. After the completion of reaction, the reaction mixture was concentrated to remove off solvent and then give compound IV.
(d) Compound IV was added into purified water and stirred until complete dissolution. At a temperature controlled below 20°C, sodium carbonate aqueous solution was dropwise added to adjust pH to 8. Then, dichloromethane was added to extract three times, the aqueous phase is separated and removed out. The organic phase was cooled down to a temperature below 5°C, and a solution of oxalic acid in methanol was dropwise added. The mixture was stirred to crystalize, to give compound V (81% yield). The characterization data of the obtained compound are the same as those in Example 1.

### Example 5

Compare the properties of the desired salts of compound V, includes but not limits to as herein oxalate or phosphate, to hydrochloride. Conduct the stability test under the condition of temperature 40 °C and humidity 75%, measure the chemical purity by HPLC and observe the physical properties. The results of stability test are shown in table 2. The results show that the solid hygroscopicity of compound V can be improved by changing its salt forms, further avoid the deterioration of compound V during transport and storage.

**Table 2. Comparison of compound V salt forms**

| Salt forms | Initial purity | 40°C/75% placement for 1 day | | |
|---|---|---|---|---|
| | | Purity (%) | Hygroscopicity | Appearance |
| Oxalate (1: 2) | 99.1 | 99.0 | Almost none | White powder |
| Phosphate (1: 2) | 98.5 | 96.1 | Slight hygroscopicity | White to light yellow powder |
| Hydrochloride (1: 2) | 95.1 | 87.5 | Strong hygroscopicity | Yellow solid (severely solidified) |

## Claims

1. A method for preparing formula V, Methyl (S)-2-amino-3-[4-(2,3-dimethylpyridin-4-yl)-phenyl]-propionate diacid salt, **characterized in that**, the method comprises the following steps:
step (a): reacting compound I with bis(pinacolato)diboron to give compound II;
step (b): reacting compound II with 4-bromo-L-phenylalanine to give compound III;
step (c): subjecting compound III to esterification to give compound IV;
step(d): after salt dissociation of compound IV, mixing it in free form with organic acid or inorganic acid to form salt and then give compound V after purification;
with the following scheme: 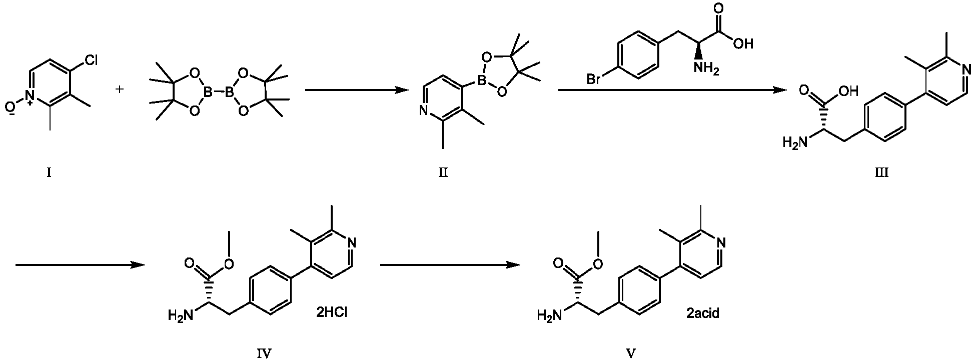
wherein the compound V salt form is selected from the salt group consisting oxalate, L-tartrate, malate, succinate, maleate, citrate, phosphate, sulfate, hydrobromide, preferably oxalate and phosphate.

2. The method according to claim 1, **characterized in that**, in step (a), catalyst A is added, and the catalyst A is a palladium catalyst alone or a mixed system of palladium catalyst and organophosphorus ligand, wherein the palladium catalyst is selected from the group consisting of Pd₂(dba)₃, PdCl₂(PPh₃)₂, and Pd(OAc)₂, and the organophosphorus ligand is one, two or more selected from the group consisting of PCy₃, PPh₃, n-Bu₃P, and P(OMe)₃; and/or in step (b), catalyst B is added, and the catalyst B is a palladium catalyst alone or a mixed system of palladium catalyst and organophosphorus ligand, wherein the palladium catalyst is selected from the group consisting of Pd(OAc)₂, Pd₂(dba)₃, PdCl₂(PPh₃)₂, PdCl₂dppf, and Pd(PPh₃)₄, and the organophosphorus ligand is one, two or more selected from the group consisting of Ph₂P(CH₂)₂PPh₂(dppe), Ph₂P(CH₂)₃PPh₂(dppp), PCy₃, n-Bu₃P, P(OMe)₃, PPh₃; and/or the total molar amount of the catalysts used in steps (a) and (b) is 0.1^{~}5% of that of the total reaction system, wherein the molar amount of the catalyst used in step (a) is 0.1^{~}1% of that of compound I, and the molar amount of the catalyst used in step (b) is 1^{~}5% of that of 4-bromo-L-phenylalanine.

3. The method according to claim 1, **characterized in that**, in step (a), an organic acid salt is added, wherein the organic acid salt is one, two or more selected from the group consisting of potassium acetate, sodium acetate, potassium oxalate, sodium oxalate, sodium citrate, potassium citrate, L-potassium tartrate, L-sodium tartrate, potassium malate, sodium malate, potassium succinate, sodium succinate, potassium maleate, sodium maleate; and/or in step (b), after the adjustment of pH, alkaline reagent B is added, wherein the alkaline reagent B is one, two or more selected from the group consisting of sodium carbonate, potassium carbonate, cesium carbonate, lithium carbonate, barium hydroxide, and potassium phosphate; and/or the solvent used in step (b) is one, two or more selected from the group consisting of ethanol, n-propanol, n-butanol, tetrahydrofuran, 1,4-dioxane, toluene, and xylene.

4. The method according to claim 3, **characterized in that**, in step (a), compound I, organic acid salt and bis(pinacolato)diboron are fed in a molar ratio in the range of 1:2:2^{~}1:4:3; and/or in step (b), 4-bromo-L-phenylalanine and compound II are fed at a molar ratio in the range of 1:1.5^{~}1:2.5, and preferably 1:2.

5. The method according to claim 1, **characterized in that**, in step (c), methanol serves as both solvent and reaction reagent; and/or oxalyl chloride or thionyl chloride is added; and/or the reaction temperature is controlled in the range of 40^{~}70°C, and preferably in the range of 55^{~}65°C.

6. The method according to claim 1, **characterized in that**, an alkaline aqueous solution is added to the product of step (c) to adjust pH, and then the mixture is extracted with solvent; aqueous phase is separated and removed out, and organic phase is concentrated to give free base of compound IV; and/or the extraction solvent is one selected from the group consisting of ethyl acetate, dichloromethane, isopropyl acetate, butyl acetate or 2-methyltetrahydrofuran.

7. The method according to claim 1, **characterized in that**, in step (d), the solution of the free base of compound IV is mixed homogenously with solvent, and then a solution of organic acid or inorganic acid is added; the mixture is stirred to form salt and crystalize, and then compound V is obtained.

8. The method according to claim 7, **characterized in that**, in step (d), the solvent is one, two or more selected from the group consisting of dichloromethane, acetone, isopropanol, acetonitrile, and tetrahydrofuran; and/or the organic acid or inorganic acid is one selected from the group consisting of oxalic acid, L-tartaric acid, malic acid, succinic acid, maleic acid, citric acid, phosphoric acid, sulfuric acid, and hydrobromic acid, and preferably oxalic acid or phosphoric acid; and/or the organic acid or inorganic acid is dissolved in acetone, methanol or ethanol.

9. Use of the methyl (S)-2-amino-3-[4-(2,3-dimethylpyridin-4-yl)-phenyl]-propionate diacid salt obtained according to any of claims 1-8 for preparing (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid dihydrochloride.
